# EUROPÄISCHE PATENTSCHRIFT

(11) **EP 0 492 500 B1**
(45) Veröffentlichungstag und Bekanntmachung des Hinweises auf die Patenterteilung: **10.04.1996**
(21) Anmeldenummer: 91121936.8
(22) Anmeldetag: 20.12.1991
(51) Int. Cl.: C12N 15/30, C12N 15/63, A61K 39/015, C07K 14/00

(54) **Protektive Plasmodium falciparum Hybridproteine, die Teilsequenzen der Malaria-Antigene HRPII und SERP enthalten**
Protecting plasmodium falciparum hybrid proteins, containing partial sequences of malaria antigens HRPII and SERP
Protéines hybrides, protectrices de plasmodium falciparum qui contiennent des séquences partielles des antigènes de paludisme HRPII et SERP

(30) Priorität: 24.12.1990 DE 4041836
(43) Veröffentlichungstag der Anmeldung: 01.07.1992
(73) Patentinhaber: BEHRINGWERKE Aktiengesellschaft, D-35001 Marburg (DE)
(72) Erfinder: Knapp, Bernhard, Dr., W-3550 Marburg (DE); Hundt, Erika, Dr., W-3550 Marburg (DE); Küpper, Hans, Dr., W-3550 Marburg (DE); Enders, Burkhard, Dr., W-3550 Marburg (DE)

(56) Entgegenhaltungen:
- EP-A- 0 245 862
- EP-A- 0 275 196
- EP-A- 0 283 882
- EP-A- 0 315 085
- EP-A- 0 322 712
- WO-A-88/02757
- BEHRING INSTITUT MITTEILUNGEN 0, Band 88, 1991, Medizinische Verlagsges., Marburg (DE), 4th Internatl. Erlangen Workshop on Molecular Aspects of Immunological Host-Parasite-Interaction, Erlangen (DE), 27-29 September 1990; B. KNAPP et al., Seiten 147-156
- IMMUNOLOGY LETTERS, Bd. 25, Nr. 1-3, August 1990, Elsevier Science, Amsterdam (NL); C. ROUSSILHON et al., Seiten 149-154
- SCIENCE, Band 240, 03 Juni 1988, Aaad, Lancaster, PA (US); A. CRISANTI et al., Seiten 1324-1326
- PROCEEDINGS OF THE NATL. ACADEMY OF SCIENCES USA, Band 83, August 1986, Washington, DC (US); T.E.WELLEMS et al., Seiten 6065-6069

## Beschreibung

Die Erfindung betrifft Hybridproteine bestehend aus Teilsequenzen der Malaria-Antigene HRPII und SERP. Die HRPII Sequenz erwies sich bereits als protektiv im Affenmodell (EP-A2-0 315 085), wobei in der vorliegenden Erfindung ebenso wie in dem vorausgehenden Schutzversuch ein C-terminaler Bereich von 189 Aminosäuren eingesetzt wurde. SERP wurde mit Hilfe eines Antiserums gegen eine protektive Proteinbande identifiziert (EP-A1-0 283 882); die verwendete Teilsequenz (Aminosäuren (AS) 631-892 bzw. 630-764) enthält mindestens zwei T-Zell Epitope (Roussilhon et al. (1990), Immunol. Letters 25, 149-154). In bevorzugten Ausführungsformen werden weitere T-Zell Epitope enthaltende Bereiche anderer P. falciparum Proteine wie das "Merozoite Surface Antigen I" (MSA I oder auch "195 kd Antigen"), Aminosäuren von 100 bis 300 eingebaut (Crisanti et al. (1988), Science 240, 324-326). Allen Hybridkomponenten gemeinsam ist die Eigenschaft, daß sie hochkonservierte Peptidsequenzen darstellen. Durch Impfung mit vorgenannten Hybridproteinen wird Schutz gegen eine Malaria-Infektion erzeugt.

Im Hinblick auf die Bedeutung von Malaria als einer der am weitesten verbreiteten Tropenkrankheiten ist die Bereitstellung eines wirksamen Impfstoffs von größter Bedeutung. Impfstoffe können gegen Sporozoiten, Merozoiten oder Gametozyten von Plasmodien (P.) gerichtet sein, wobei hier Antigene des asexuellen Blutstadiums (Merozoiten) von P. falciparum ausgewählt wurden.

Die Nukleotid- und Proteinsequenz von SERP ist in der Europäischen Patentanmeldung EP-A1-0 283 882 als diejenige für das "140kd Protein" beschrieben. HRPII ist in der Europäischen Patentanmeldung EP-A2-0 315 085 beschrieben; die Nukleinsäuresequenz und das davon kodierte Protein sind in Tab. 2 ebendort enthalten. Eine Teilsequenz von MSA I schließlich ist in der Europäischen Patentanmeldung EP-A1-0254862 unter der Bezeichnung Antigen 31-1 bzw. Teilprotein des 195 kd Antigens (siehe Fig. 1 ibidem) beschrieben.

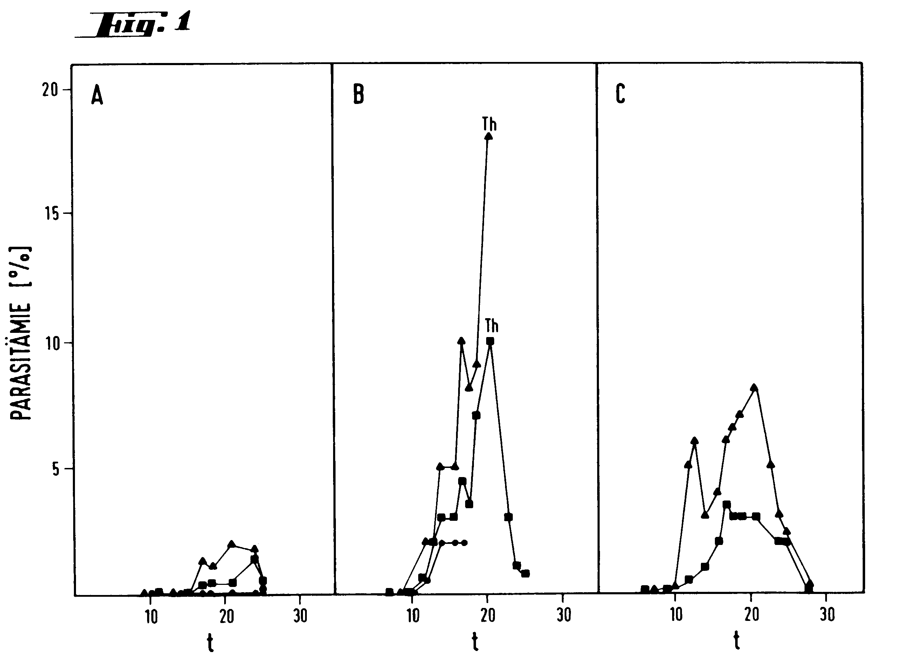

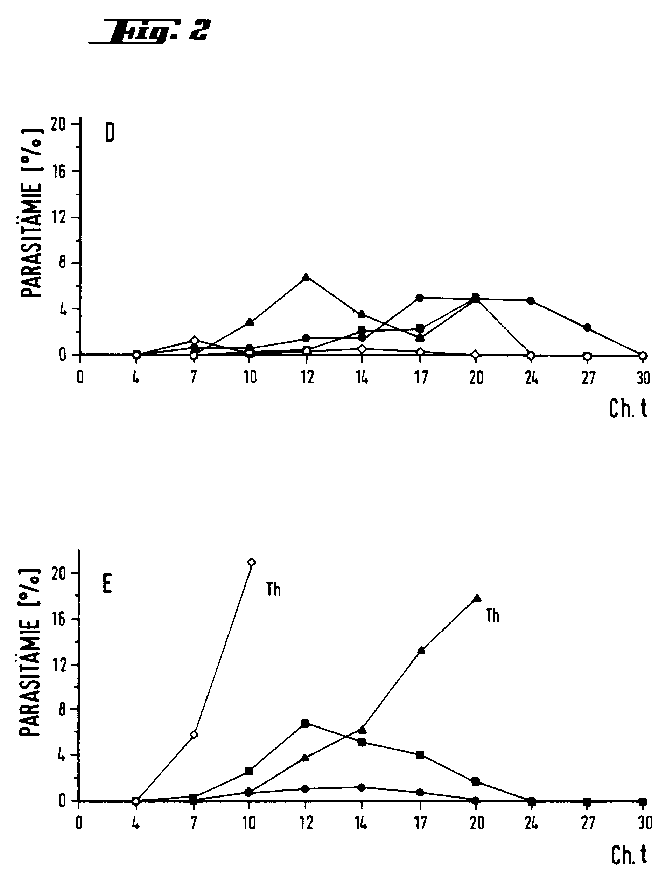

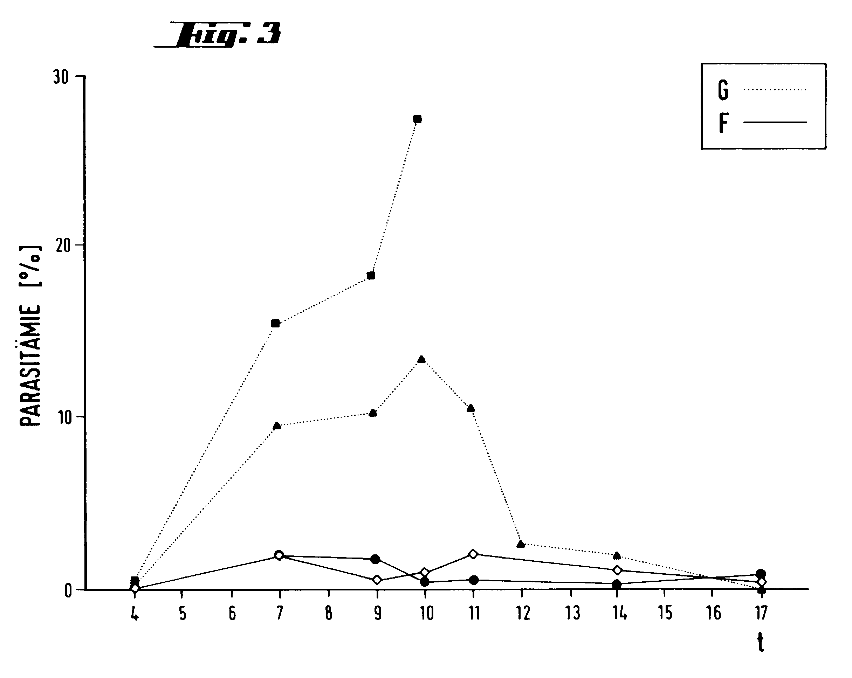

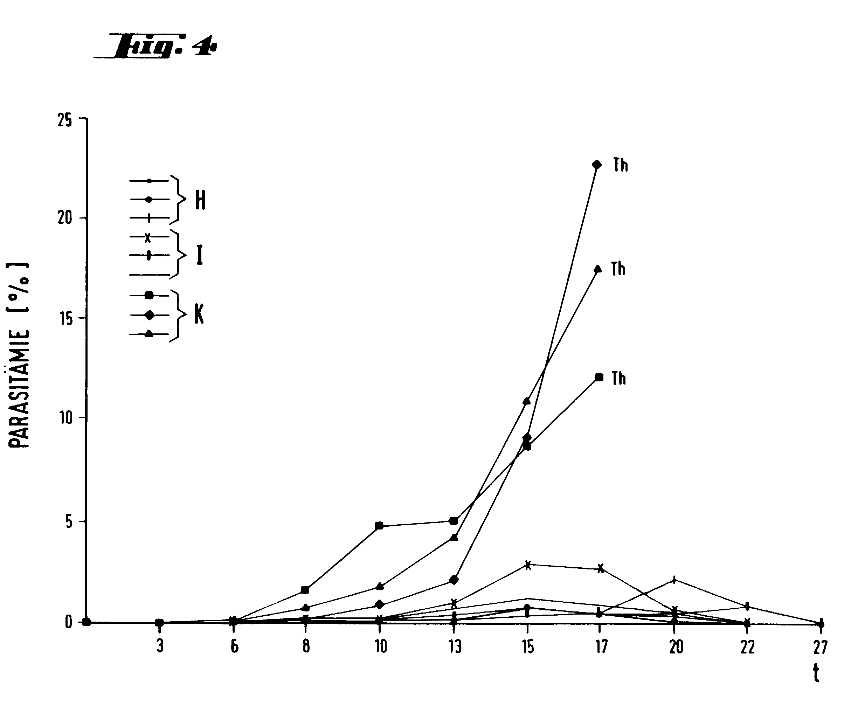

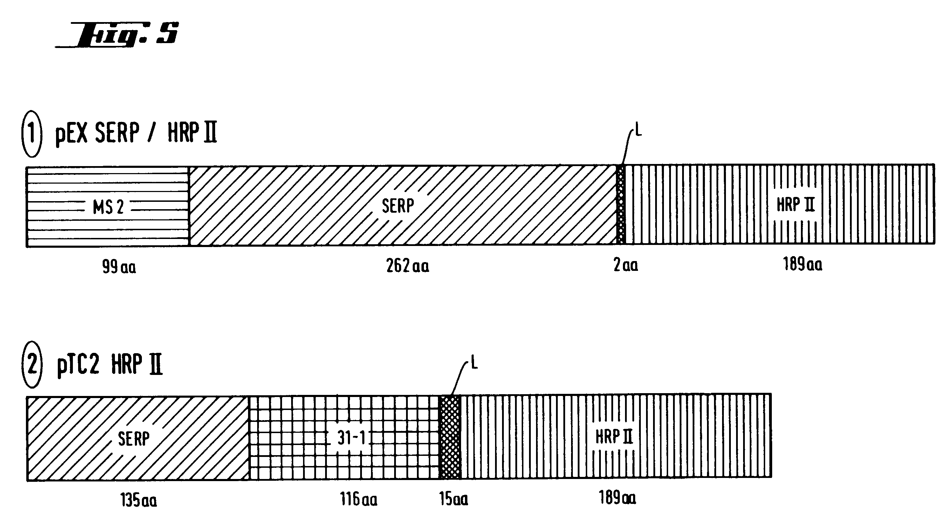

Es stellte sich überraschenderweise heraus, daß die erfindungsgemäßen Hybridproteine einen gegenüber den jeweiligen Einzelproteinen verstärkten protektiven Effekt zeigen (Fig. 1-4). Beispielhaft für die erfindungsgemäßen Hybridproteine wurden die Expressionsprodukte zweier Konstrukte eingesetzt:
1. MS2/SERP/HRP II (enthält SERP AS 631-892) und
2. SERP/MSA I/HRP II (enthält MSA I AS 140-254 und SERP AS 630-764),
die schematisch in Fig. 5 dargestellt sind. Die Fusionsproteine beinhalten dabei mindestens zwei T-Zell Epitope. Zusätzlich zu dem synergistischen protektiven Effekt bei Einsatz der erfindungsgemäßen Hybridproteine ist das günstigere Reinigungsverfahren solcher bi-, tri- oder oligopartiter Hybridkonstruktionen zu sehen, da in nur einem einzigen Reinigungsverfahren mehrere hochwirksame Bestandteile einer Malariavaccine gewonnen werden. Eine Mehrkomponenten-Vakzine verringert zusätzlich das Risiko, daß der Impfstoff durch Mutation des Erregers unwirksam wird.

In den nachfolgenden Beispielen ist die Erfindung weiter beschrieben, wobei die vorgestellten Konstrukte MS2/SERP/HRP II und SERP/MSA I/HRP II die Erfindung erläutern aber nicht beschränken. Die Erfindung ist schließlich durch die Ansprüche spezifiziert.

### Beispiele:

### Beispiel 1:

### Beschreibung der Ausgangsplasmide

Zur Konstruktion der Hybridantigene MS2/SERP/HRPII und SERP/MSA I/HRPII wurden folgende Plasmide verwendet:
- pUC-SERP: der Vektor pUC 18 enthält ein 5.6 kb genomisches XbaI-Fragment, das das komplette SERP Gen trägt (EP-A1-0 283 882; B. Knapp et al. (1989) Mol. Biochem. Parasitol. 32, 73 - 84).
- pUC-HRPII: der Vektor pUC 18 enthält ein 600 bp Fragment, das für den C-terminalen Teil des HRPII Proteins codiert (EP-A2-0 315 085; B. Knapp et al (1988) Behring Inst. Mitt. 82, 349 - 359).
- pEx-31-1lrd: der Vektor pEX 31b enthält ein 600 bp Fragment, das für den N-terminalen Teil des Antigens MSA I unter Deletion einer repetitiven Sequenz codiert (EP-A1-0 254 862).
- pEX31: Expressionsvektor, der unter der Kontrolle des PL-Promoters des lambda-Phagen 99 N-terminale Aminosäuren der Polymerase des MS2-Bakteriophagen, an welche ein Fremdprotein fusioniert werden kann, exprimiert (K. Strebel et al. (1986) J. Virol. 57, 983 - 991).
- pTRC99: Expressionsvektor, der unter der Kontrolle des tac Promoters ein Fremdprotein fusionsfrei exprimieren kann (E. Amann et al. (1988) Gene 69, 301 - 315).

### Beispiel 2:

### Konstruktion des Hybridantigens MS2/SERP/HRPII

Das Plasmid pUC-SERP wurde mit den Restriktionsenzymen EcoRI und PstI verdaut, wobei ein 787 bp Fragment des SERP Genes nach herkömmlichen Methoden (J. Sambrook et al. (1989) Molecular Cloning: A Laboratory Manual, 2nd edn., Cold Spring Harbor Laboratory, Cold Spring Harbor, NY, USA) isoliert werden konnte. Dieses DNA-Fragment wurde zwischen die EcoRI und PstI Restriktionsstellen des Vektors pEX31b ligiert. POP 2136 Zellen (Fa. Stratagene), die das CI 537 Gen in ihrem Chromosom tragen, wurden transformiert und Einzelkolonien wurden auf ihre Expression der parasitenspezifischen DNA-Sequenz als MS2-Polymerase Fusionsprotein nach Hitzeinduktion gemäß beschriebenen Methoden (B. Knapp et al., 1988 a. a. o.) untersucht. Einzelkolonien, die das DNA-Fragment in den pEX31b Vektor eingebaut tragen, exprimieren ein 40kD MS2-Polymerase-Fusionsprotein, welches die Aminosäuren 631 - 892 des SERP-Antigens enthält. Diese Region enthält zwei T-Zell Epitope zwischen den Aminosäurepositionen 640 und 700 (C. Roussilhon et al. (1990) a.a.O.) und eine Region von Aminosäureposition 745 bis 787, die homolog zur Consensus-Sequenz von Cystein Proteinasen ist (Higgins et al. (1989), Nature 340, 604).

In einem zweiten Schritt wurde das Plasmid pUC-HRPII, welches am 5' und am 3'Ende zu der insertierten DNA eine PstI Stelle trägt, mit dem Restriktionsenzym HindIII verdaut und anschließend unter partiellen Verdauungsbedingungen mit dem Enzym PstI inkubiert (0.05 Einheiten für 10 min. bei 37°C), um ein DNA-Fragment von 600 bp zu isolieren, welches eine PstI-Stelle am 5'Ende und eine HindIII-Stelle (partielle Verdauung) oder eine PstI-Stelle (komplette Verdauung) am 3' Ende trägt. Dieses DNA-Fragment wurde zwischen die PstI und die HindIII Stelle des Plasmids pEX31b welches das 787 bp SERP-Fragment trägt, ligiert. Dabei stimmt der Leserahmen des einligierten HRPII-Fragmentes mit dem des insertierten SERP-Fragmentes überein. Nach Transformation von POP 2136 Zellen wurden individuelle Klone bei 28°C in LB-Medium, dem 100 µg/ml Ampicillin zugesetzt wurde, angezüchtet und die Plasmid DNA jedes Klons durch Restriktionsenzym-Verdauung nach bekannten Methoden (J. Sambrook et al., a.a.o.) analysiert. Klone, die die gewünschten DNA-Fragmente insertiert hatten, wurden nach beschriebenen Methoden (B. Knapp et al., 1988) zur Expression angeregt und durch SDS-Polyacrylamidgelelektrophorese analysiert. Die Proteine wurden dabei entweder mit Coomassie Blau gefärbt oder auf Nitrocellulose transferriert, um sie mit Antikörpern, die gegen rekombinante Teilsequenzen von SERP (B. Knapp et al., 1989 a.a.o.) und HRPII (B. Knapp et al., 1988 a.a.o.) gerichtet sind, zu untersuchen. Im Gegensatz zu der von der Aminosäuresequenz abgeleiteten Größe von 62 kD hat das Fusionsprotein ein Molekulargewicht von 75 kD. Die Ursache hierfür liegt in dem bereits beschriebenen ungewöhnlichen Wanderungsverhalten des HRPII Anteils (B. Knapp et al., 1988 a.a.o.). Das Fusionsprotein enthält Teile von drei verschiedenen Proteinen: 99 Aminosäuren der MS2-Polymerase (AS 1 - 99), 262 Aminosäuren von SERP (AS 100 - 361) und 189 Aminosäuren von HRPII (AS 364 - 552). Die Aminosäurepositionen 362 und 363 werden durch eine Linkerregion codiert, der aus der Klonierungsprozedur stammt. Dieses Hybridgen benutzt das TAA Codon des HRPII Fragmentes als sein natürliches Stopcodon. Das von diesem Hybridgen codierte Protein reagiert in der Western Blot Analyse mit Antiseren, die für SERP bzw. für HRPII spezifisch sind. Dies bestätigt, daß das Hybridprotein antigene Determinanten beider Malariaproteine enthält.

Aus 5 l induzierter Bakterienkultur wurde das Hybridprotein durch schrittweises Steigern der Harnstoffkonzentration nach beschriebenen Methoden (B. Knapp et al., 1988 a.a.o.) partiell gereinigt. Das Hybridprotein ging dabei in 7 M Harnstoff in Lösung und war gegen 2 M Harnstoff dialysierbar - die minimale Harnstoffkonzentration, um das Protein in Lösung zu halten. Mit dem partiell gereinigten Hybridprotein wurden Kaninchen immunisiert. Antikörper, die gegen das Hybridprotein gerichtet sind, reagieren sowohl mit SERP als auch mit HRPII zu etwa gleichen Anteilen, wie durch Western Blot Analyse mit P. falciparum Schizont Proteinen nachgewiesen wurde.

### Beispiel 3:

### Konstruktion des Hybridantigens SERP/MSA I/HRPII

Die Oligonukleotide p1 (5'-CGTCCCATGGAATTCTTACAAATTATTGAAGAT-3', NcoI Restriktionsstelle am 5' Ende, komplementär zu den Basen 2641 - 2667 des SERP Gens) und p2 (5'-TCCTTCGCTATTCACATAATTACCATAACCAACAATATTAACTGCATG-3', komplementär zu den Basen 2993 - 3045 des SERP Gens), sowie 10 ng der Plasmid-DNA pUC-SERP wurden in einer "Polymerase-Ketten-Reaktion" (PCR) eingesetzt. Die PCR wurde nach einem Standardprotokoll unter Benutzung des Gen Amp™ Kits (Fa. Perkin Elmer Cetus) durchgeführt. Daraus resultierte ein ca. 400 bp Fragment, das für 135 Aminosäuren des SERP Antigers kodiert (AS 630 bis 764) - eine Region, welche zwei T-Zell Epitope des SERP-Antigens enthält (C. Roussilhon et al., 1990 a.a.o.).

10 ng der Plasmid-DNA pEX-31-1lrd wurde in Kombination mit den Oligonukleotiden p3 (5'-GGTAATTATGTGAATAGCGAAGGAGAACTCTTTGATTTAACCAATCATATG-3'; Nukleotide 1 - 18 sind komplementär zu den Basen 3022 - 3045 des SERP Gens und Nukleotide 19- 45 sind komplementär zu den Basen 226 - 252 der Partialsequenz 31-1 lrd von MSA I) und p4 (5'-GGGGTCGACGGATCCGGTACCAAGCTTACTTCCTTCAATTAATTCATTTATATTTGC-3', komplementär zu den Basen 538 - 567 der Partialsequenz 31-1 lrd von MSA I mit SalI, BamHI, KpnI und HindIII Restriktionsstellen am 5' Ende) mit Hilfe der PCR amplifiziert. Das daraus resultierende 360 bp Fragment kodiert für 115 Aminosäuren des MSA I Antigens (AS 140-254). Es handelt sich dabei um einen unter verschiedenen P. falciparum Stämmen hoch konservierten Bereich, der zwei T-Zell Epitope trägt (Crisanti et al., 1988, a.a.o.).

Jeweils 150 ng der DNA-Fragmente aus der 1. und der 2. PCR wurden in Kombination mit den Oligonukleotiden p1 und p4 nach der Methode von Hortom et al. (Gene 77, 61-68, 1989) einer dritten PCR unterworfen. Das amplifizierte 760 bp Fragment wurde mit den Restriktionsenzymen NcoI und SalI verdaut und zwischen die NcoI und SalI Stellen des Vektors pTRC99 unter Benutzung von Standardmethoden (J. Sambrook et al., 1989 a.a.o.) insertiert. Dadurch wurde dem pTRC Vektor ein Hybridgen einkloniert, das für Teile des SERP und des MSA I Antigens kodiert. Das Startcodon ist dabei Teil des SERP Antigens, d. h. dieses Konstrukt besitzt N-terminal keine Fusion mit einer malariaunspezifischen Sequenz. Das fusionierte Gen kodiert insgesamt für mindestens 4 T-Zell-Epitope.

Um zu überprüfen, ob dieses Hybridgen tatsächlich exprimiert wird, wurde das konstruierte Plasmid in kompetente DH5α E. coli Zellen transformiert und wie von Amann et al., (1988 a.a.o.) beschrieben durch Isopropylthiogalaktosid (IPTG) induziert. Die induzierten Zellen wurden durch Zentrifugation abgeerntet und das Proteinmuster durch SDS-PAGE analysiert. Die Proteine wurden durch Coomassie-Blau gefärbt oder auf Nitrocellulose transferriert, welche mit Antikörpern gegen SERP und gegen das MSA I Antigen inkubiert wurde . Das Proteinmuster zeigt, daß nach Induktion ein 28 kD Protein in hoher Ausbeute exprimiert wird. Dieses Protein ist ein Hybridprotein, bestehend aus 135 Aminosäuren von SERP und 115 Aminosäuren des MSA I Antigens. Es reagiert im Western Blot mit Antiseren gegen SERP und gegen das MSA I Antigen positiv. Das Plasmidkonstrukt, das aus dem pTRC Vektor und dem Hybridgen besteht, wurde als Plasmid pTC bezeichnet.

Um Restriktionsfragmente weiterer P. falciparum Antigene unabhängig von ihrem Leserahmen einklonieren zu können, wurde der Polylinker des pTC Vektors durch den Polylinker der Vektoren pTRC99A,B,C (Amann et al., 1988, a.a.o.) ersetzt. Dazu wurde der Vektor pTC mit HindIII verdaut und anschließend die HindIII Restriktionsstelle mit T4-DNA Polymerase nach bekannten Methoden (J. Sambrook et al., 1989, a.a.o.) am 3' Ende mit Nukleotiden aufgefüllt. Anschließend wurde dieser Vektor mit ScaI verdaut, wobei ein 830 bp Fragment anfiel, welches gelelektrophoretisch von der Vektor DNA getrennt wurde. Die Vektor DNA wurde aus dem Gel eluiert. Gleichzeitig wurden die Vektoren pTRC99A,B und C mit NcoI verdaut und anschließend wurden die 5' überstehenden Enden mit T4-DNA-Polymerase aufgefüllt. Nach Restriktion der drei Vektoren mit ScaI fiel jeweils ein 890 bp Fragment an, das gelelektrophoretisch von der Vektor-DNA getrennt wurde. Dieses 890 bp NcoI-ScaI Fragment der Vektoren pTRC99A,B und C wurde in den pTC-Vektor-Rest einkloniert. Daraus resultierten die Plasmide pTC1, pTC2 und pTC3, die den Polylinker des pTRC Vektors in den drei verschiedenen Leserahmen tragen.

Die Plasmid DNA pUC-HRPII wurde mit den Restriktionsendonucleasen BamHI und HindIII verdaut. Daraus resultierte ein DNA Fragment von 610 bp, das gelelektrophoretisch gereinigt wurde. Gleichzeitig wurde der Vektor pTC2 mit den gleichen Restriktionsenzymen behandelt und dephosphoryliert. Das HRPII spezifische DNA Fragment wurde nach beschriebenen Methoden (J. Sambrook et al, 1989, a.a.o.) in den pTC2 Vektor einligiert. Nach Transformation von DH5α-Zellen wurden individuelle Kolonien bei 37°C auf LB-Agarplatten angezüchtet, die 100 µg/ml Ampicillin enthalten. Plasmid-DNA von einigen dieser Kolonien wurde isoliert und durch Restriktion mit BamHI und HindIII auf Insertion des HRPII Fragments untersucht. Positive Kolonien wurden über Nacht bei 37°C geschüttelt und anschließend für 2 Stunden mit 1 mM IPTG induziert. Nach Abernten der Bakteriensuspension durch Zentrifugation wurde das Proteinmuster durch SDS -PAGE analysiert. Dabei wurden die Proteine entweder direkt mit Coomassie Blau gefärbt oder sie wurden auf Nitrocellulose transferiert und mit Antikörpern gegen SERP, gegen MSA I und gegen HRPII untersucht. Die Bakterien exprimieren eine zusätzliche Proteinbande von 62 kD, welche im Western Blot mit Antiseren gegen die drei Malariaproteine SERP, MSA I und HRPII reagiert. Dieses Hybridantigen ist dreiteilig und trägt 135 Aminosäuren von SERP, 115 Aminosäuren des MSA I Antigens und 189 Aminosäuren des HRPII Antigens. Die Aminosäuren der Positionen 251 bis 265 werden von der Linker-Region kodiert.

### Beispiel 4:

### Schutzversuche im Aotus-Modell

a. Einzelkomponenten HRPII und 31-1 lrd (Fragment, das die verwendete MSA I Region enthält)
   9 Aotusaffen (1000 - 1500 g Körpergewicht, männliche und weibliche Tiere aus der Zucht der Behringwerke AG) wurden randomisiert und in 3 Gruppen zu je 3 Tieren aufgeteilt (Gruppe A, B, C).
   Alle 3 Tiere der Gruppe A wurden in Abständen von 3 Wochen dreimal mit jeweils 100 µg eines MS2/HRPII Fusionsproteins (gelöst in PBS) subkutan immunisiert. Als Adjuvans diente eine 10%ige Zumischung von 50% Al(OH)₃ + 45% Lecithin + 5% Saponin zum Antigen.
   Die 3 Tiere der Gruppe B wurden nach dem gleichen Schema mit MS2-Fusionsprotein immunisiert, das die Aminosäuren 26-66 und 106-258 von MSA I enthält.
   3 Tiere der Infektions-Kontrollgruppe (Gruppe C) erhielten ebenfalls nach o. g. Schema jeweils eine Injektion aus PBS + Adjuvans ohne Antigenkomponente.
b. Einzelkomponente SERP
   8 Aotusaffen (1000 - 1500 g Körpergewicht, männliche und weibliche Tiere aus der Zucht der Behringwerke AG) wurden randomisiert und in 2 Gruppen zu je 4 Tieren aufgeteilt (Gruppe D, E).
   Alle 4 Tiere der Gruppe D wurden in Abständen von 3 Wochen dreimal mit einer Kombination der SERP Regionen AS 68 - 184 und AS 631 - 892, beide als MS2 Fusionsproteine exprimiert, (100 µg pro Fusionsprotein und Dosis, gelöst in PBS/3M Harnstoff) subkutan immunisiert. Als Adjuvans diente eine 10%ige Zumischung von 50% Al(OH)₃ + 45% Lecithin + 5% Saponin zum Antigen.
   4 Tiere der Infektions-Kontrollgruppe (Gruppe E) erhielten ebenfalls nach o. g. Schema jeweils eine Injektion aus Lösungsmittel + Adjuvans ohne Antigenkomponente.
c. Hybridprotein MS2/SERP/HRPII
   4 Aotusaffen (1000 - 1500 g Körpergewicht, männliche und weibliche Tiere aus der Zucht der Behringwerke AG) wurden randomisiert und in 2 Gruppen zu je 2 Tieren aufgeteilt (Gruppe F, G).
   Die 2 Tiere der Gruppe F wurden in Abständen von 3 Wochen dreimal mit jeweils 100 µg des Hybridproteins (gelöst in PBS/3M Harnstoff) subkutan immunisiert. Als Adjuvans diente eine 10%ige Zumischung von Polyalphaolefin zum Antigen.
   2 Tiere der Infektions-Kontrollgruppe (Gruppe G) erhielten ebenfalls nach o. g. Schema jeweils eine Injektion aus PBS/3M Harnstoff + Adjuvans ohne Antigenkomponente.
d. Hybridproteine MS2/SERP/HRPII und SERP/MSAI/HRPII
   9 Aotusaffen (1000 - 1500 g Körpergewicht, männliche und weibliche Tiere aus der Zucht der Behringwerke AG) wurden randomisiert und in 3 Gruppen zu je 3 Tieren aufgeteilt (Gruppe H, I, K).
   Alle 3 Tiere der Gruppe H wurden in Abständen von 3 Wochen dreimal mit jeweils 100 µg des Hybridproteins MS2/SERP/HRPII (gelöst in PBS/3M Harnstoff) subkutan immunisiert. Als Adjuvans diente eine 10%ige Zumischung von Polyalphaolefin zum Antigen. Die 3 Tiere der Gruppe I wurden nach dem gleichen Schema mit dem Hybridprotein SERP/MSAI/HRPII immunisiert.
   Die 3 Tiere der Infektions-Kontrollgruppe (Gruppe K) erhielten ebenfalls nach o. g. Schema jeweils eine Injektion aus PBS/3M-Harnstoff + Adjuvans ohne Antigenkomponente.
   Alle Tiere der Gruppen A-K wurden 1 Woche nach der letzten Immunisierung splenektomiert und erhielten ca. 1 Woche später eine Belastungsinfektion von 5 x 10⁶ (A-G) bzw. 2 x 10⁶ (H-K) parasitierten Erythrozyten ex vivo (P. falciparum Palo Alto).

### Legende:

Fig. 1 - 4: Schutzversuche im Aotus Modell (Beispiel 4)
P. % = % Parasitämie
t = Tage nach Infektion
Th = Therapie
Ch. = Challenge (Infektion mit P. falciparum, Belastung der Impfung)

- Fig. 1:: Impfung mit Einzelkomponenten HRPII (Gruppe A) und MSA I (Gruppe B); Kontrolle = Gruppe C
- Fig. 2:: Impfung mit Einzelkomponente SERP (Gruppe D); Kontrolle = Gruppe E
- Fig. 3:: Impfung mit Hybridprotein MS2/SERP/HRPII (Gruppe F); Kontrolle = Gruppe G
- Fig. 4:: Impfung mit Hybridproteinen MS2/SERP/HRPII (Gruppe H) und SERP/MSAI/HRPII (Gruppe I); Kontrolle = Gruppe K
- Fig. 5:: 1) beschreibt schematisch das Konstrukt aus MS2, SERP und HRPII (Plasmid pEX SERP/HRP II), wobei ein Linker von 2 Aminosäuren zwischen SERP und HRPII eingebaut ist.
2) beschreibt schematisch entsprechend das Plasmid pTC2 HRP II.
aa = Aminosäuren

## Patentansprüche (Patentansprüche für folgende(n) Vertragsstaat(en): AT, BE, CH, DE, DK, FR, GB, IT, LI, LU, NL, SE)

1. Hybridproteine aus Plasmodium falciparum, die mindestens zwei T-Zell-Epitope von SERP sowie eine Teilsequenz von HRPII tragen.

2. Hybridproteine nach Anspruch 1, dadurch gekennzeichnet, daß der Bereich AS 630 - AS 892 von SERP und 189 C-terminale AS von HRPII umfaßt sind.

3. Hybridproteine nach Anspruch 1, dadurch gekennzeichnet, daß zusätzlich ein MSAI-Anteil mit mindestens 2 T-Zell-Epitopen eingebaut ist.

4. Verfahren zur Herstellung von Hybridproteinen nach Anspruch 1, 2 oder 3, dadurch gekennzeichnet, daß die für besagte Hybridproteine kodierenden Sequenzen in geeigneten Expressionssystemen exprimiert und die resultierenden Expressionsprodukte isoliert werden.

5. Hybridproteine nach Anspruch 1, 2 oder 3 als Vaccine.

6. Vaccine, die Hybridproteine nach Anspruch 1, 2 oder 3 und ein geeignetes Adjuvans enthält.

## Patentansprüche (Patentansprüche für folgende(n) Vertragsstaat(en): ES, GR)

1. Verfahren zur Herstellung von Hybridproteinen aus Plasmodium falciparum, die mindestens zwei T-Zell-Epitope von SERP sowie eine Teilsequenz von HRPII tragen, dadurch gekennzeichnet, daß die für besagte Hybridproteine kodierenden Sequenzen in geeigneten Expressionssystemen exprimiert und die resultierenden Expressionsprodukte isoliert werden.

2. Verfahren zur Herstellung von Hybridproteinen nach Anspruch 1, dadurch gekennzeichnet, daß der Bereich AS 630 - AS 892 von SERP und 189 C-terminale AS von HRPII umfaßt sind.

3. Verfahren zur Herstellung von Hybridproteinen nach Anspruch 1 oder 2, dadurch gekennzeichnet, daß zusätzlich ein MSAI-Anteil mit mindestens 2 T-Zell-Epitopen eingebaut ist.

4. Verwendung von Hybridproteinen, hergestellt nach einem Verfahren nach mindestens einem der Ansprüche 1 - 3, als Vaccine.

5. Verwendung von Hybridproteinen nach Anspruch 4, dadurch gekennzeichnet, daß die Vaccine zusätzlich ein geeignetes Adjuvans enthält.

## Claims (Claims for the following Contracting State(s): AT, BE, CH, DE, DK, FR, GB, IT, LI, LU, NL, SE)

1. A hybrid protein from Plasmodium falciparum, which carries at least two T-cell epitopes of SERP and a part-sequence of HRPII.

2. A hybrid protein as claimed in claim 1, which includes the region AA 630 - AA 892 of SERP and 189 C-terminal AA of HRPII.

3. A hybrid protein as claimed in claim 1, wherein an MSAI portion with at least 2 T-cell epitopes is additionally incorporated.

4. A process for the preparation of hybrid proteins as claimed in claim 1, 2 or 3, which comprises expressing the sequences coding for said hybrid proteins in suitable expression systems, and isolating the resulting expression products.

5. A hybrid protein as claimed in claim 1, 2 or 3 as vaccine.

6. A vaccine which contains a hybrid protein as claimed in claim 1, 2 or 3 and a suitable adjuvant.

## Claims (Claims for the following Contracting State(s): ES, GR)

1. A process for the preparation of hybrid proteins from Plasmodium falciparum, which carry at least two T-cell epitopes of SERP and a part-sequence of HRPII, which comprises expressing the sequences coding for said hybrid proteins in suitable expression systems, and isolating the resulting expression products.

2. The process for the preparation of hybrid proteins as claimed in claim 1, which include the region AA 630 - AA 892 of SERP and 189 C-terminal AA of HRPII.

3. The process for the preparation of hybrid proteins as claimed in claim 1 or 2, wherein an MSAI portion with at least 2 T-cell epitopes is additionally incorporated.

4. The use of hybrid proteins, prepared by a process as claimed in at least one of claims 1 - 3, as vaccine.

5. The use of hybrid proteins as claimed in claim 4, wherein the vaccine additionally contains a suitable adjuvant.

## Revendications (Revendications pour l'(les) Etat(s) contractant(s) suivant(s): AT, BE, CH, DE, DK, FR, GB, IT, LI, LU, NL, SE)

1. Protéines hybrides dérivées de Plasmodium falciparum, portant au moins deux épitopes de cellules T de SERP ainsi qu'un fragment de séquence de HRPII.

2. Protéines hybrides selon la revendication 1, caractérisées en ce qu'elles comprennent la zone AA 630- AA 892 de SERP et la partie C-terminale de 189 AA de HRPII.

3. Protéines hybrides selon la revendication 1, caractérisées en ce que un fragment de MSA I est également incorporé avec au moins deux épitopes de cellules T.

4. Procédé pour la préparation de protéines hybrides selon la revendication 1, 2 ou 3, caractérisé en ce que les séquences codant pour lesdites protéines hybrides sont exprimées dans des systèmes d'expression appropriés et les produits d'expression résultants sont isolés.

5. Protéines hybrides selon la revendication 1, 2 ou 3, en tant que vaccin vaccin.

6. Vaccin contenant les protéines hybrides selon la revendication 1, 2 ou 3, ainsi qu'un adjuvant approprié.

## Revendications (Revendications pour l'(les) Etat(s) contractant(s) suivant(s): ES, GR)

1. Procédé pour la préparation de protéines hybrides dérivées de Plasmodium falciparum, portant au moins deux épitopes de cellules T de SERP ainsi qu'un fragment de séquence de HRPII, caractérisé en ce que les séquences codant pour lesdites protéines hybrides sont exprimées dans des systèmes d'expression appropriés et les produits d'expression résultants sont isolés.

2. Procédé pour la préparation de protéines hybrides selon la revendication 1, caractérisé en ce qu' elles comprennent la zone AA 630 - AA 892 de SERP et la partie C-terminale de 189 AA de HRPII.

3. Procédé pour la préparation de protéines hybrides selon la revendication 1 ou 2, caractérisé en ce qu'un fragment de MSA I est également incorporé avec au moins deux épitopes de cellules T.

4. Utilisation des protéines hybrides préparées selon un procédé d'au moins une des revendications 1 à 3, en tant que vaccin.

5. Utilisation de protéines hybrides selon la revendication 4, caractérisée en ce que le vaccin contient en outre un adjuvant approprié.
